# EUROPEAN PATENT APPLICATION

(11) **EP 4 049 720 A1**
(43) Date of publication of application: **31.08.2022**
(21) Application number: 22158282.8
(22) Date of filing: 23.02.2022
(51) Int. Cl.: A61N 1/378

(54) **REDUCED POWER CONSUMPTION FOR ELECTRICAL STIMULATION THERAPY**

(30) Priority: 24.02.2021 US 202163153075 P; 03.02.2022 US 202217649880
(71) Applicant: Medtronic, Inc., Minneapolis, Minnesota 55432 (US)
(72) Inventor: Torgerson, Nathan A., Minneapolis, 55432 (US); Zenisek, Todd D., Minneapolis, 55432 (US)
(74) Representative: Zimmermann & Partner Patentanwälte mbB

(57) **Abstract**

Devices, systems, and techniques are described for adjusting therapy parameters defining electrical stimulation therapy. An example system includes a stimulation generator comprising a voltage stack configured to provide a stack voltage based on a multiplier of a battery voltage and processing circuitry. The processing circuitry receives a first set of parameter values that use a first stack voltage of the voltage stack to provide a first electrical stimulation defining a first therapy. The processing circuitry also determines, based on a second stack voltage lower than the first stack voltage, a second set of parameter values that define a second electrical stimulation, the second set of parameters defining a lower amplitude of electrical stimulation. Additionally, the processing circuitry controls the stimulation generator to deliver the second electrical stimulation according to the second set of parameter values using the second stack voltage of the voltage stack.

## Description

This application claims the benefit of U.S. Provisional Patent Application No. 63/153,075, filed February 24, 2021, the entire contents of which is incorporated herein by reference.

### TECHNICAL FIELD

The disclosure relates to medical devices and, more particularly, to medical devices that deliver electrical stimulation therapy.

### BACKGROUND

Medical devices may be used to treat a variety of medical conditions. Medical electrical stimulation devices, for example, may deliver electrical stimulation therapy to a patient via electrodes. Electrical stimulation therapy may include stimulation of nerve, muscle, or brain tissue, or other tissue within a patient. An electrical stimulation device may be fully implanted within the patient. For example, an electrical stimulation device may include an implantable electrical stimulation generator and one or more implantable leads carrying electrodes. The electrical stimulation device may comprise a leadless stimulator. In some cases, implantable electrodes may be coupled to an external electrical stimulation generator via one or more percutaneous leads or fully implanted leads.

Medical electrical stimulators may be used to deliver electrical stimulation therapy to patients to relieve a variety of symptoms or conditions such as chronic pain, tremor, Parkinson's disease, depression, epilepsy, urinary or fecal incontinence, pelvic pain, sexual dysfunction, obesity, or gastroparesis. An electrical stimulator may be configured to deliver electrical stimulation therapy via leads that include electrodes proximate to the spinal cord, pelvic nerves, gastrointestinal organs, peripheral nerves, or within the brain of a patient. Stimulation proximate the spinal cord and within the brain are often referred to as spinal cord stimulation (SCS) and deep brain stimulation (DBS), respectively.

### SUMMARY

In general, the disclosure describes devices and techniques for controlling an electrical stimulation device using alternative stimulation parameter values. Simulation generators within the electrical stimulation device use therapy parameters values that define the electrical characteristics of electrical stimulation delivered to a patient via implantable electrodes. These stimulation parameters may at least partially define electrical stimulation delivered via the electrode and may include, e.g., a current amplitude, a voltage amplitude, an electrical stimulation pulse count, a frequency, a pulse width, electrode combination, etc. An initial set of therapy parameters values may be determined to provide effective therapy to a patient. Generally, the initial set of therapy parameters are determined by a physician or medical technician and may use feedback from the patient to provide effective therapy.

As described below, an electrical stimulation device may store multiple sets of therapy parameters in memory, which may include the initial set of therapy parameters and additional sets of parameters. The additional set(s) of therapy parameters may provide effective therapy, that may or may not be similar to the therapy provided by the initial set of therapy parameters, while conserving power stored in a battery of the electrical stimulation device. For example, the additional sets of therapy parameters stored or determined by the system may preserve effective therapy while requiring a lower voltage stack setting of the stimulation generator.

In some examples, the electrical stimulation device may determine the additional set(s) of therapy parameters by changing an amplitude and pulse width of the simulation signal while maintaining a substantially (e.g., ± 10%) equivalent charge density or volume of activation. In some examples, the electrical stimulation device may determine the additional set(s) of therapy parameters by changing an amplitude and pulse width of the simulation signal according to a strength-duration curve on which the initial set of therapy parameters lies. For example, a lower amplitude and longer pulse width as compared to the initial set of parameters may result in a similar therapy that consumes less energy. In some examples, from time to time, the electrical stimulation device monitors characteristics (e.g., lead impedance, etc.) of the stimulation generation circuitry and/or electrodes that may be indicative that the initial set of therapy parameters would be more effective. In some such examples, the electrical stimulation device may switch to driving the electrodes with the initial set of therapy parameters. The device may additionally or alternatively adjust other stimulation parameters, such as pulse frequency, in order to achieve more efficient energy usage for stimulation.

An example system includes a stimulation generator comprising a voltage stack configured to provide a stack voltage based on a multiplier of a battery voltage and processing circuitry. The processing circuitry receives a first set of parameter values that use a first stack voltage of the voltage stack to provide a first electrical stimulation defining a first therapy. The processing circuitry also determines, based on a second stack voltage lower than the first stack voltage, a second set of parameter values that define a second electrical stimulation, the second set of parameters defining a lower amplitude of electrical stimulation. Additionally, the processing circuitry controls the stimulation generator to deliver the second electrical stimulation according to the second set of parameter values using the second stack voltage of the voltage stack.

An example method includes receiving a first set of parameter values that use a first stack voltage of a voltage stack to provide a first electrical stimulation defining a first therapy. The method also includes determining, based on a second stack voltage lower than the first stack voltage, a second set of parameter values that define a second electrical stimulation to provide the first therapy, the second set of parameter values defining a lower amplitude of electrical stimulation. Additionally, the method includes controlling a stimulation generator to deliver the second electrical stimulation according to the second set of parameter values using the second stack voltage of the voltage stack.

An example computer readable medium comprising instructions that when executed, cause a system to control a stimulation generator receive a first set of parameter values that use a first stack voltage of a voltage stack to provide a first electrical stimulation defining a first therapy. The instructions further cause the system to determine, based on a second stack voltage lower than the first stack voltage, a second set of parameter values that define a second electrical stimulation to provide the first therapy, the second set of parameter values defining a lower amplitude of electrical stimulation. Additionally, the instructions cause the system to control a stimulation generator to deliver the second electrical stimulation according to the second set of parameter values using the second stack voltage of the voltage stack.

The details of one or more examples of the techniques of this disclosure are set forth in the accompanying drawings and the description below. Other features, objects, and advantages of the techniques will be apparent from the description and drawings, and from the claims.

### BRIEF DESCRIPTION OF DRAWINGS

FIG. 1 is a conceptual diagram illustrating an example therapy system that includes an electrical stimulator coupled to a stimulation lead, in accordance with various techniques of this disclosure.
FIG. 2 is a block diagram illustrating the example programmer of FIG. 1 in further detail.
FIG. 3 is a block diagram illustrating the example electrical stimulator of FIG. 1 in further detail.
FIG. 4 is a block diagram illustrating an example of the electrical stimulation generation circuitry of the electrical stimulator of FIG. 3 in further detail.
FIG. 5 is a flowchart of an example method to control delivery of electrical stimulation in accordance with the techniques of this disclosure.
FIG. 6 illustrates example strength-duration curves.
FIGS. 7A and 7B are conceptual diagrams illustrating screens of an example user interface in accordance with the techniques of the disclosure.
FIGS. 8A and 8B are conceptual diagrams illustrating example battery efficiencies for different pulse rates at various pulse widths.
FIG. 9 illustrates an example user interface that provides estimated longevity information for different pulse rates of electrical stimulation.

Like reference characters refer to like elements throughout the figures and description

### DETAILED DESCRIPTION

Devices, systems, and techniques are described to determine alternative sets of therapy parameters for delivering electrical stimulation. Electrical stimulation devices include simulation generators that drive electrodes to provide therapeutic stimulation to a patient. These electrical stimulation devices also include batteries (rechargeable or non-rechargeable) that provide electrical power to drive the electrodes. Generally, stimulation generation circuitry requires voltages greater than the voltage of the battery to generate desired electrical stimulation amplitudes. To provide this increased voltage, simulation generators can include voltage stacks (sometimes referred to as "voltage ladders" and/or "voltage pumps"). Voltage stacks trade efficiency for multiples of the available battery voltage. Voltage stacks provide these multiples of battery voltage in discrete steps (e.g., controlled by a frequency driving the voltage stack). For example, at a first setting, the voltage stack may provide a 1.25 battery voltage multiplier, and at a second setting, the voltage stack may provide a 1.50 battery voltage multiplier. In such an example, if the electrical stimulation device includes a 3-volt (V) battery, the voltage stack may deliver 3.75V and 4.50V respectively. However, higher voltage stack settings are less energy efficient. The stimulation generator of the electrical stimulation device may be able to deliver several different amplitude levels of electrical stimulation at the same setting of the voltage stack (e.g., the same voltage multiplier). When the electrical stimulation device needs to increase the setting to jump up the voltage stack in order to achieve the amplitude of the electrical stimulation requested by the stimulation parameter value, some energy of that voltage multiplier is wasted because not all of the voltage is required to generate the electrical signal having an amplitude at the low end of that setting of the voltage stack.

As described below, an electrical stimulation device determines and stores multiple sets of parameters for therapeutic stimulation. These parameters may at least partially define electrical stimulation delivered via an electrode and may include a current amplitude (e.g., in milliamps (mA), etc.) and a pulse width (e.g., in microseconds (µS), etc.). The electrical stimulation device includes an initial set of parameters to provide effective therapy to a patient. Generally, the initial set of parameters are set by a physician or medical technician with feedback from the patient to determine what set of parameters provide effective therapy. Subsequently, an electrical stimulation device and/or a programming device, may determine a secondary set of parameters (e.g., different amplitude, pulse width, pulse frequency, etc.) that provide the effective therapy while driving a voltage stack at a lower setting (e.g., at a lower step frequency, etc.) to reduce overall energy consumption.

In an example scenario, the initial set of parameters may use a current amplitude that requires an input voltage of 4.00V. To obtain this voltage, the voltage stack may be driven to provide a 1.5 multiplier to a 3V battery included in the electrical stimulation device (e.g., 4.50V). The next lower available multiplier may be 1.25, which would result in a voltage of 3.75V with the 3V battery. As can be seen from this example, some energy will be lost when using the 4.50V input voltage with 1.5 multiplier. Due to this extra energy expended, the electrical stimulation device and/or the programming device may determine a second set of parameters such that the current amplitude of the second set of parameters requires a voltage input of 3.75V or less while achieving a similar therapy (e.g., a similar current density).

In some examples, the electrical stimulation device and/or the programming device may change (e.g., lengthen, etc.) a pulse width parameter to provide the effective therapy when lowering the amplitude of the electrical stimulation to use a lower voltage multiplier setting. In some examples, the electrical stimulation device and/or the programming device sets the secondary set of parameters so that the electrodes provide a substantially equivalent (e.g., ± 10%) charge (e.g., total charge or charge density). In some examples, the electrical stimulation device and/or the programming device sets the secondary set of parameters based on a strength duration curve that associates an amplitude setting with a pulse width setting (e.g. based on an analysis of patient data, etc.). The strength duration curve defines a relationship between the settings for typical nerve fibers of the patient. In some examples, the electrical stimulation device and/or the programming device sets the secondary set of parameters so that the electrodes provide a substantially equivalent charge density based on, for example, a VOA model that models a volume of activation (e.g., volume of neural activation (VNA), volume of tissue activated (VTA), a stimulation field model (SFM), and/or an electrical stimulation field (ESF), etc.) based on stimulation parameters that define an intersection between predicted activated tissue and non-activated tissue. Although this disclosure generally describes deep brain stimulation, these techniques may also be applicable to other therapies, such as spinal cord stimulation (SCS), peripheral nerve stimulation (PNS), sacral nerve stimulation, pelvic floor stimulation, or any other type of electrical stimulation therapy.

In some examples, the electrical stimulation device monitors characteristics of leads of the electrodes (e.g., lead impedance) indicative that the electrical stimulation device should return to the initial set of parameters. For example, some temporary conditions, such as dehydration, can cause this increased impedance. Under such conditions, the electrical stimulation device may switch to the initial set of parameters. In such a manner, the electrical stimulation device may provide effective therapy while increasing battery longevity.

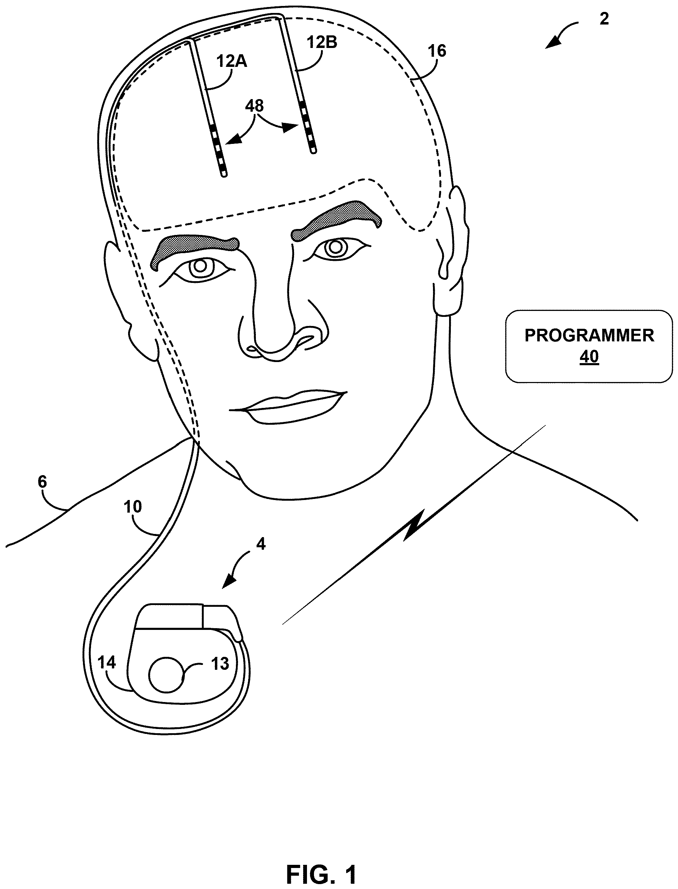

FIG. 1 is a conceptual diagram illustrating example therapy system 2 that includes electrical stimulator 4 coupled to stimulation lead 10, in accordance with various techniques of this disclosure. Therapy system 2 may be configured to deliver stimulation therapy to patient 6. Patient 6 ordinarily, but not necessarily, will be a human. Generally, therapy system 2 includes electrical stimulator 4 (e.g., an implantable medical device (IMD)) that delivers electrical stimulation to patient 6 via one or more electrodes disposed on stimulation lead extension 10. Electrical stimulator 4 delivers stimulation therapy, e.g., in the form of electrical stimulation, via one or more electrodes 48 disposed along one or more medical leads 12A and 12B which connect to lead extension 10. For purposes of description electrodes 48 are described as being implantable electrodes. However, the example techniques are not limited to implantable electrodes.

Electrodes 48 may be deployed on one or more medical leads, such as medical leads 12A and 12B, and in some cases on a housing electrode. The electrical stimulation may be in the form of controlled current pulses or voltage pulses, or substantially continuous current or voltage waveforms. A stimulation program may define various parameters of the pulses or waveforms. In some examples, the pulses or waveforms are defined by an amplitude parameter and a pulse width parameter. The pulses or waveforms may be delivered substantially continuously or in bursts, segments, or patterns, and may be delivered alone or in combination with pulses or waveforms defined by one or more other stimulation programs. In some examples, one or more of the electrodes may be located on a housing 14 of the electrical stimulator 4. In addition, implantable electrodes may be deployed on a leadless stimulator.

In some examples, electrical stimulator 4 may deliver, for example, deep brain stimulation (DBS) or cortical stimulation (CS) therapy to patient 6 via the electrodes carried by lead 12. Although FIG. 1 shows a particular stimulation environment (e.g., DBS), the techniques of this disclosure are not so limited, and electrical stimulator 4 may deliver stimulation therapy to other parts of patient 6, such as the spinal cord of patient 6 as described in U.S. Patent No. 8,560,080, entitled, "PROGRAMMING TECHNIQUES FOR CONTROLLING RATE OF CHANGE OF ELECTRICAL STIMULATION THERAPY," by Goetz et al, and U.S. Patent No. 8,996,123, entitled, "MANAGING ELECTRICAL STIMULATION THERAPY BASED ON VARIABLE ELECTRODE COMBINATIONS," by Goetz et al, the contents of which are incorporated by reference herein in their entirety. For example, other electrical stimulation systems may be configured to deliver electrical stimulation to gastrointestinal organs, pelvic nerves or muscle, peripheral nerves, or other stimulation sites. In addition, although FIG. 1 shows a fully implantable electrical stimulator 4, techniques described in this disclosure may be applied to external stimulators having electrodes deployed via percutaneous leads.

In the example illustrated in FIG. 1, electrical stimulator 4 is implanted in a clavicle region of patient 6. Electrical stimulator 4 generates programmable electrical stimulation (e.g., a current or voltage waveform or current or voltage pulses) and delivers the stimulation via a medical lead 10 carrying an array of stimulation electrodes 48. In general, delivery of electrical stimulation using controlled current pulses will be described in this disclosure for purposes of illustration. In some cases, electrical stimulator may include multiple leads. In the example of FIG. 1, a distal end of lead 10 is bifurcated and includes two leads 12A and 12B (collectively "leads 12"). Leads 12A and 12B each include a set of electrodes forming part of the array of electrodes 48. In various examples, leads 12A and 12B may each carry four, eight, or sixteen electrodes. In FIG. 1, each lead 12A, 12B carries four electrodes, configured as ring electrodes at different axial positions near the distal ends of the leads 12.

In other examples, one or more of leads 12A or 12B may include a different array of electrodes. For example, lead 12A may include electrodes at different positions around the perimeter of the lead. In one example, three, four, or more electrodes may be at the same axial position but different circumferential positions around the lead. These electrodes at different circumferential positions may be referred to as "segmented electrodes" because they represent "segments" of a ring around the lead. These electrodes at the same axial position may be referred to as being disposed at the same "level" of the lead. In some examples, a lead may include one or more levels of multiple electrodes and may include one or more complete ring (or cylindrical electrodes) in addition to the one or more levels of multiple electrodes. An example lead may include, from proximal to distal end of the lead, a proximal ring electrode, a first level of three electrodes, a second level of three electrodes, and a distal ring electrode. In other examples, a lead may include four levels of electrodes, where each level has two, three, four, or more electrodes. The electrodes may be circumferentially aligned or offset between levels.

FIG. 1 further depicts a housing electrode 13. Housing electrode 13 may be formed integrally with an outer surface of hermetically-sealed housing 14 of electrical stimulator 4, or otherwise coupled to housing 14. In one example, housing electrode 13 may be described as an active, non-detachable electrode on electrical stimulator 4. In some examples, housing electrode 13 is defined by an uninsulated portion of an outward facing portion of housing 14 of electrical stimulator 4. Other divisions between insulated and uninsulated portions of housing 14 may be employed to define two or more housing electrodes. In some examples, housing electrode 13 comprises substantially all of housing 14, one side of housing 14, a portion of housing 14, or multiple portions of housing 14.

In some examples, electrical stimulator 4 may be coupled to one or more leads which may or may not be bifurcated. In such examples, the leads may be coupled to electrical stimulator 4 directly or via a common lead extension (such as lead extension 10) or separate lead extensions. A proximal end of lead extension 10 may be coupled to a header on electrical stimulator 4. Conductors in the lead body may electrically connect stimulation electrodes located on leads 12 to electrical stimulator 4. Lead extension 10 traverses from the implant site of electrical stimulator 4 along the neck of patient 6 before coupling to leads 12A and 12B. Leads 12A and 12B continue to traverse to the brain 16 of patient 6. In some examples, leads 12A and 12B may be implanted within the right and left hemispheres, respectively, in order to deliver electrical stimulation to one more regions of brain 16.

Leads 12A, 12B may be implanted within a desired location of brain 16 through respective holes in the cranium of patient 6. Leads 12A, 12B may be placed at any location within brain 16 such that the electrodes located on leads 12A, 12B are capable of providing electrical stimulation to targeted tissue. The electrodes of leads 12A, 12B are shown as ring electrodes. In some examples, the electrodes of leads 12A, 12B may have different configurations. For example, the electrodes of leads 12A, 12B may have a complex electrode array geometry that is capable of producing shaped electrical fields. The complex electrode array geometry may include multiple electrodes (e.g., partial ring or electrode "segments") around the perimeter of each leads 12A, 12B. In some examples, leads 12 may have shapes other than elongated cylinders as shown in FIG. 1. For example, leads 12 may be paddle leads, spherical leads, bendable leads, or any other type of shape effective in treating patient 6. In addition, the electrodes may be electrode pads on a paddle lead, circular electrodes surrounding the body of a lead, conformable electrodes, cuff electrodes, segmented electrodes, or any other type of electrodes capable of forming unipolar, bipolar, multi-polar, etc. electrode configurations.

In some examples, electrical stimulator 4 delivers stimulation according to a group of programs at a given time. Each program of such a program group may include respective values for each of a plurality of therapy parameters. The therapy parameters may include, e.g., one of a current or a voltage amplitude, a pulse width, a pulse shape, a pulse rate or pulse frequency, a number of pulses, or an electrode configuration (e.g., electrode combination and polarity). Electrical stimulator 4 may interleave pulses or other signals according to the different programs of a program group. In such examples, programmer 40 may be used to create programs, and assemble the programs into program groups. In some examples, programmer 40 may be used to adjust stimulation parameters of one or more programs of a program group, and select a program group as the current program group to control delivery of stimulation by electrical stimulator 4.

Generally, system 2 delivers stimulation therapy to patient 6 in the form of constant current or voltage waveforms or constant current or voltage pulses. The shapes of the pulses may vary according to different design objectives, and may include ramped or trapezoidal pulses, sinusoidal or otherwise curved pulses, stepped pulses having two or more discrete amplitudes, closely spaced pairs of pulses, and biphasic (positive and negative aspects within a single pulse) or monophasic (only positive or only negative aspects within a single pulse) variations of any of the above. In the case of current-based stimulation, electrical stimulator 4 regulates current that is sourced or sunk by one or more electrodes, referred to as regulated electrodes. In some examples, one or more of the electrodes may be unregulated. In such configurations, the housing electrode and/or a lead electrode may be the unregulated electrode.

A source current may refer to a positive current that flows out of an electrode (anode), whereas a sink current may refer to a negative current that flows into an electrode (cathode). Regulated source currents may sum to produce a greater overall source current (e.g., currents from a plurality of source currents sum together to generate the overall source current). Likewise, regulated sink currents may sum to produce a greater overall sink current (e.g., currents from a plurality of sink currents sum together to generate the overall sink current). Regulated source and regulated sink currents may partially or entirely cancel one another, producing a net difference in the form of a net source current or sink current in the case of partial cancellation. In some examples, an unregulated current path can source or sink current approximately equal to this net difference. In some examples, regulated source and sink currents may be substantially balanced.

In some example implementations (e.g., bipolar/multipolar arrangements), one or more electrodes 48 may be configured to act as anodes and source current while one or more different electrodes 48 may be configured to act as cathodes and sink current. In another example implementation (e.g., unipolar arrangements), housing electrode 13 may be configured to act as an anode and source current while one or more electrodes 48 on one or more leads are configured to act as cathodes and sink current. The techniques of this disclosure may be implemented using, e.g., unipolar arrangements or bipolar/multipolar arrangements.

Therapy system 2 may include a programmer 40, such as an external programmer operated by a clinician or patient. In some examples, a programmer 40 may be a handheld computing device that permits a clinician to program stimulation therapy for patient 6 via a user interface. For example, using programmer 40, the clinician may specify stimulation parameters for use in delivery of stimulation therapy. Programmer 40 may support telemetry with electrical stimulator 4 to download programs and, optionally, upload operational or physiological data stored by electrical stimulator 4. Programmer 40 may also include a display and input keys to allow patient 6 or a clinician to interact with programmer 40 and electrical stimulator 4. In this manner, programmer 40 provides patient 6 with a user interface for control of the stimulation therapy delivered by electrical stimulator 4. For example, patient 6 may use programmer 40 to start, stop or adjust electrical stimulation. In particular, programmer 40 may permit patient 6 to adjust stimulation parameters of a program, such as duration, current or voltage amplitude, pulse width, pulse shape, and pulse rate. Patient 6 may also select a program (e.g., from among a plurality of stored programs) as the present program to control delivery of stimulation by electrical stimulator 4.

In some cases, programmer 40 may be characterized as a physician or clinician programmer 40. For example, programmer 40 may include a clinician programmer if programmer 40 is primarily intended for use by a physician or clinician. In other cases, programmer 40 may be characterized as a patient programmer if programmer 40 is primarily intended for use by a patient. In general, a physician or clinician programmer may support selection and generation of programs by a clinician for use by stimulator 4, whereas a patient programmer may support, during ordinary use, adjustment and selection by a patient of such programs as allowed by the clinician and/or clinician programmer.

Whether programmer 40 is configured for clinician or patient use, programmer 40 may communicate with electrical stimulator 4 or any other computing device via wireless communication. Programmer 40, for example, may communicate via wireless communication with electrical stimulator 4 using RF telemetry techniques known in the art. Programmer 40 may also communicate with another programmer or computing device via a wired or wireless connection using any of a variety of local wireless communication techniques, such as radio frequency (RF) communication according to the 802.11 or Bluetooth specification sets, infrared communication according to the Infrared Data Association (IrDA) specification set, or other standard or proprietary telemetry protocols. Programmer 40 may also communicate with another programming or computing device via exchange of removable media, such as magnetic or optical disks, or memory cards or sticks. Further, programmer 40 may communicate with electrical stimulator 4 and other programming devices via remote telemetry techniques known in the art, communicating via a local area network (LAN), wide area network (WAN), public switched telephone network (PSTN), or cellular telephone network, for example.

A user, such as a clinician or patient 6, may interact with a user interface of programmer 40 to program electrical stimulator 4. In accordance with various techniques described in this disclosure, programmer 40 may be used to receive user input, via the user interface specifying one or more therapy parameters for defining electrical stimulation therapy delivered by electrical stimulator 4. Programmer 40 may control electrical stimulator 4 to cause electrical stimulator 4 to deliver electrical stimulation therapy in accordance with the specified therapy parameters, as described in more detail below, or otherwise program stimulator 4. Programming of electrical stimulator 4 may refer generally to the generation and transfer of commands, programs, or other information to control the operation of electrical stimulator 4. For example, programmer 40 may transmit programs, parameter adjustments, program selections, group selections, or other information to control the operation of electrical stimulator 4. In addition, programming of stimulator 4 may include receiving, via programmer 40, user input indicating a target stimulation zone and controlling the electrical stimulator to transition electrical stimulation from an initial stimulation zone to the target stimulation zone via a sequence of one or more intermediate stimulation zones.

Electrical stimulator 4 and programmer 40 may communicate via cables or a wireless communication, as shown in FIG. 1. Programmer 40 may, for example, communicate via wireless communication with electrical stimulator 4 using RF telemetry techniques. Programmer 40 may also communicate with other programmers using any of a variety of local wireless communication techniques, such as RF communication according to the 802.11 or Bluetooth^{™} specification sets, infrared communication (e.g., according to the IrDA standard), or other standard or proprietary telemetry protocols. Programmer 40 may include a transceiver to permit bi-directional communication with electrical stimulator 4.

In accordance with the techniques of the disclosure, system 2 determines and stores multiple sets of therapy parameters defining electrical stimulation therapy delivered by electrodes 48. Initially, system 2 determines an initial set of therapy parameters provide effective therapy. These initial set of parameters include an initial amplitude and an initial pulse-width. For example, as described above, system 2 may determine the initial set of parameters with assistance from a physician or medical technician and with feedback from the patient. Subsequently, system 2 determines an alternate or secondary set of therapy parameters to provide substantially similar therapy as the initial set of therapy parameters and, when used, reduce overall energy consumption of electrical stimulator 4. As used herein "target tolerance" and "substantially similar" refer to a value that is plus or minus (±) 10 percent of the original value.

System 2 may determine the secondary set of therapeutic parameters such that the amplitude parameter is less than the amplitude parameter of the initial set of therapy parameters and a lower voltage stack can be used when multiplying the available battery voltage. In some example, the amplitude parameter of the secondary set of parameters is below a threshold voltage. For example, for voltage pulses, system 2 determines therapy parameters such that the amplitude of the voltage is below the voltage threshold. As another example, to current pulses, system 2 determines therapy parameters such that the amplitude of the voltage to provide the current pulses is below the voltage threshold. As described below, the voltage threshold may be based on voltage tiers of a voltage stack of electrical stimulator 4. In some examples, the voltage threshold is the voltage provided by a voltage tier lower than the voltage tier required for the initial set of therapy parameters. For example, the voltage stack may provide 4.5V to generate the voltage for the initial set of therapy parameters. In such an example, the next lower tier that the voltage stack provides may be 3.75V. In such an example, the voltage threshold may be 3.75V As discussed more detail below, the voltage stack provides a voltage multiplier to the available batter voltage in order to achieve stimulation pulses with a desired voltage and/or current amplitude.

System 2 may change (e.g., lengthen, etc.) a pulse width parameter to provide the effective therapy when lowering the amplitude. In some examples, system 2 sets the secondary set of parameters so that the electrodes provide a substantially equivalent (e.g., ± 10%) charge (e.g., total charge or charge density). In some examples, system 2 sets the secondary set of parameters based on a strength duration curve that associates an amplitude setting with a pulse width setting (e.g. based on an analysis of patient data, etc.). The strength duration curve defines a relationship between the multiple parameters for typical nerve fibers of the patient. In some examples, system 2 sets the secondary set of parameters so that the electrodes provide, when compared to the initial set of parameter values, a substantially equivalent charge density based on, for example, a VOA model that models a volume of activation (e.g., volume of neural activation (VNA), volume of tissue activated (VTA), a stimulation field model (SFM), and/or an electrical stimulation field (ESF), etc.) based on therapy parameters that define an intersection between predicted activated tissue and non-activated tissue. While this disclosure describes an initial set of therapy parameters and a secondary set of therapy parameters, system 2 may determine more than two sets of therapy parameters to with different amplitudes to provide effective therapy in different conditions.

Electrical stimulator 4 stores in memory the initial set of therapy parameters and the secondary set of therapy parameters. From time-to-time, electrical stimulator 4 may monitor characteristics of one or more electrode combinations (e.g., impedances of respective current paths that may include lead conductors, electrodes, and tissue between electrodes) indicative that the electrical stimulator 4 should change which set of therapy parameters it is using. In some examples, electrical stimulator 4 may return to the initial set of therapy parameters. In some examples, electrical stimulator 4 to determine a new set of therapy parameters that provides effective therapy while maintaining a lower stack voltage (e.g., compared to the initial set of therapy parameters) as described below. In some examples, electrical simulator 4 may include a sensing circuit to measure impedance of electrodes 46. For example, some temporary conditions, such as dehydration, can cause this increased impedance. Under such conditions, electrical stimulator 4 may switch to a different set of therapy parameters (such as, for example, the initial set of therapy parameters, etc.). For example, electrical stimulator 4 may determine, based on the impedance of electrodes 46 and the amplitude of the secondary therapy parameters, that initial therapy parameters may be more suitable to generate the current amplitude to provide effective therapy. In such a manner, electrical stimulator 4 device may provide effective therapy while reducing power consumption (and increasing battery longevity).

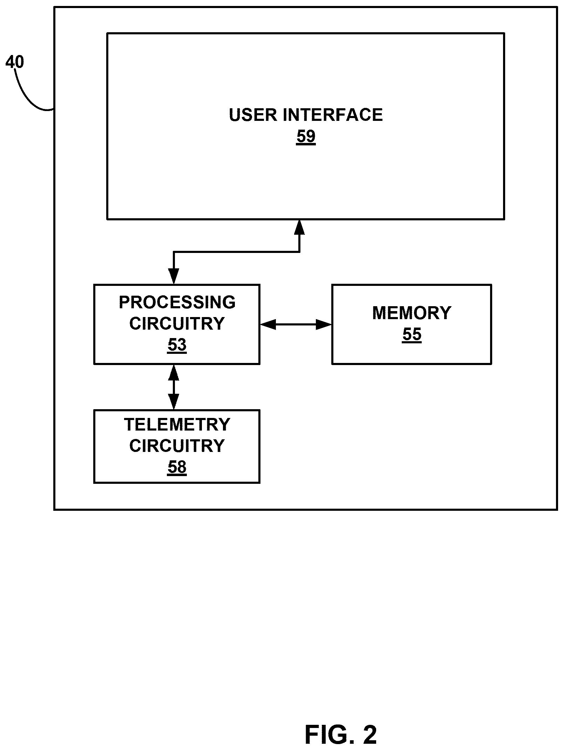

FIG. 2 is a block diagram illustrating example programmer 40 of FIG. 1 in further detail. As shown in FIG. 2, programmer 40 includes processing circuitry 53, memory 55, telemetry circuitry 58, and user interface 59. In general, processing circuitry 53 controls user interface 59, stores and retrieves data to and from memory 55, and controls transmission of data with electrical stimulator 4 through telemetry circuitry 58. Processing circuitry 53 may take the form of one or more microprocessors, controllers, digital signal processors (DSPs), application-specific integrated circuits (ASICs), field-programmable gate arrays (FPGAs), or equivalent discrete or integrated logic circuitry. The functions attributed to processing circuitry 53 herein may be embodied as software, firmware, hardware or any combination thereof.

In some examples, processing circuitry 53 determines a secondary set of therapy parameters for a patient based on an initial set of therapy parameters. Processing circuitry 53 may determine a set of therapy parameters with a lower amplitude that provide substantially similar therapy as the initial set of therapy parameters. For example, processing circuitry 53 determine a strength duration curve that associates an amplitude setting with a pulse width setting based on a relationship between the settings for typical nerve fibers of the patient. As described below, processing circuitry 53 may select an amplitude and a corresponding pulse width such that the amplitude of the secondary set of therapy parameters is in a lower voltage tier than the amplitude of the initial set of parameters. For example, memory 55 may store one or more strength duration curves from which processing circuitry 53 may select according to characteristics of the patient. In some examples, processing circuitry 53 may determine a total charge or charge density provided by the initial set of therapy parameters and select an amplitude-pulse width pair that provides substantially similar (e.g., ±10%, etc.) charge or charge density with a lower amplitude. In some examples, processing circuitry 53 determines the secondary set of therapy parameters based on VOA models of the initial set of therapy parameters. The VOA model predicts the volume of neural tissue that is activated from stimulation delivered according to the set of therapy parameters. The VOA may thus define a boundary between activated tissue and non-activated tissue. Processing circuitry 53 may select a secondary set of therapy parameters based on a VOA model when the set of parameters are predicted to substantially activate the same volume of tissue (e.g., ±10%, etc.) with substantially the same boundary (e.g., with a ±10% tolerance, etc.).

Memory 55 may store instructions that cause processing circuitry 53 to provide various aspects of the functionality ascribed to programmer 40 herein. Memory 55 may include any fixed or removable magnetic, optical, or electrical media, such as random access memory (RAM), read-only memory (ROM), compact disc ROM (CD-ROM), magnetic memory, electronically-erasable programmable ROM (EEPROM), non-volatile random access memory (NVRAM), flash memory, etc. Memory 55 may also include a removable memory portion that may be used to provide memory updates or increases in memory capacities. A removable memory may also allow patient data to be easily transferred from programmer 40 to another computing device. Memory 55 may also store information that controls operation of electrical stimulator 4

Telemetry circuitry 58 is configured to transfer data to and from electrical stimulator 4. Telemetry circuitry 58 may communicate automatically with electrical stimulator 4 at a scheduled time or when telemetry circuitry 58 detects the proximity of electrical stimulator 4. Alternatively, telemetry circuitry 58 may communicate with electrical stimulator 4 when signaled by a user through user interface 59. To support RF communication, telemetry circuitry 58 may include appropriate electronic components, such as amplifiers, filters, mixers, encoders, decoders, etc.

In some examples, programmer 40 may communicate wirelessly with electrical stimulator 4 using, for example, RF communication or proximal inductive interaction. This wireless communication is possible through the use of telemetry circuitry 58 which may be coupled to an antenna. Programmer 40 may also be configured to communicate with another computing device via wireless communication techniques, or direct communication through a wired, e.g., network, connection. Examples of local wireless communication techniques that may be employed to facilitate communication between programmer 40 and another computing device include RF communication based on the 802.11 or Bluetooth specification sets, infrared communication.

Programmer 40 includes user interface 59. A user (e.g., a clinician or patient 6) may interact with programmer 40 via user interface 59 to, for example, manually select, change or modify programs, adjust one or more therapy parameters of specific electrodes 48 or a plurality of electrodes 48, or view stimulation data. User interface 59 may comprise one or more input devices and one or more output devices. The input devices of user interface 59 may include a communication device such as a keyboard, pointing device, voice responsive system, video camera, biometric detection/response system, button, sensor, control pad, microphone, presence-sensitive screen, or any other type of device for detecting input from the user.

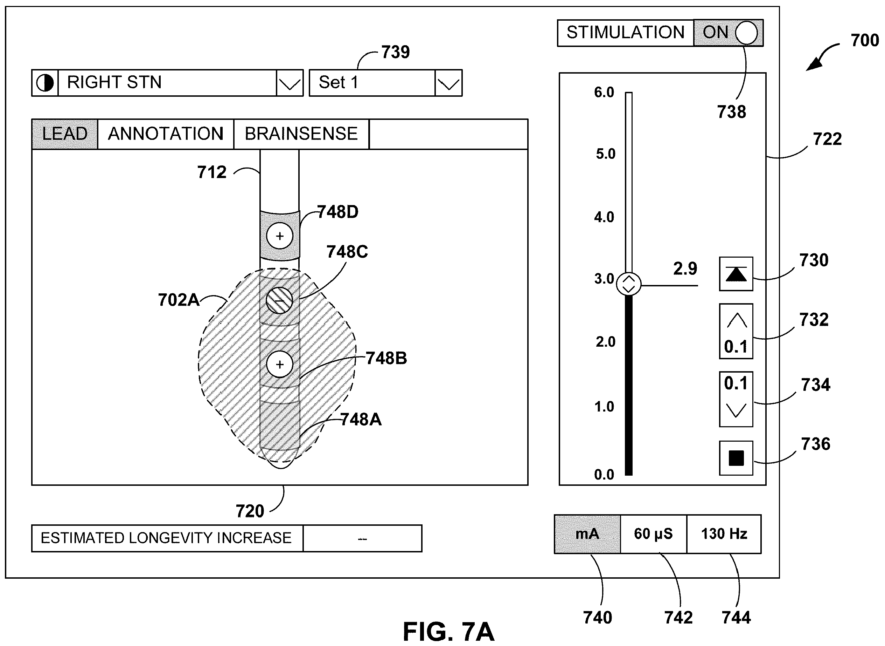

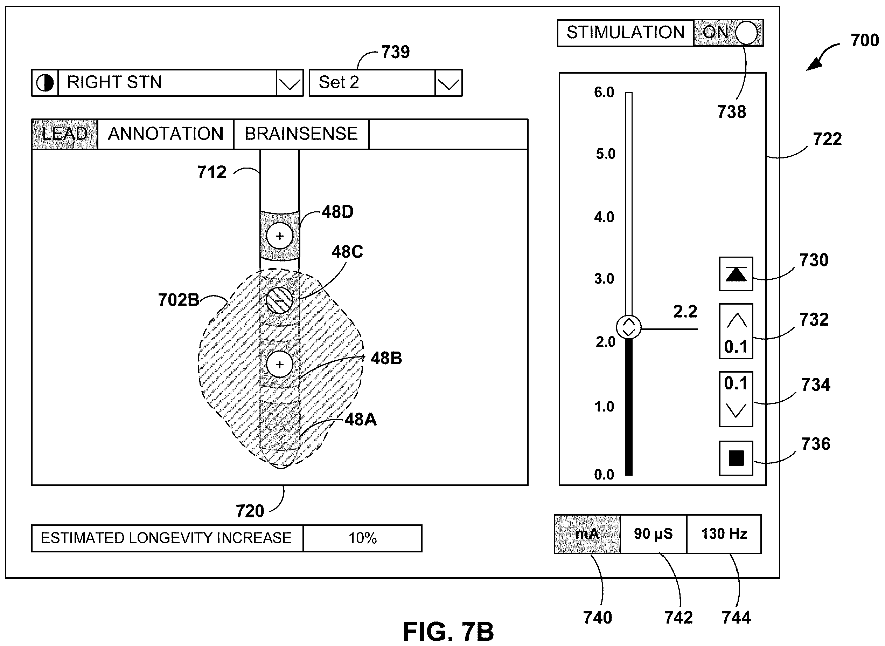

The output devices of user interface 59 may include a communication unit such as a display, sound card, video graphics adapter card, speaker, presence-sensitive screen, one or more USB interfaces, video and/or audio output interfaces, or any other type of device capable of generating tactile, audio, video, or other output. The output devices of user interface 59 may include a display device, which may function as an output device using technologies including liquid crystal displays (LCD), quantum dot display, dot matrix displays, light emitting diode (LED) displays, organic light-emitting diode (OLED) displays, cathode ray tube (CRT) displays, e-ink, or monochrome, color, or any other type of display capable of generating tactile, audio, and/or visual output. In other examples, the output devices of user interface 59 may produce an output to a user in another fashion, such as via a sound card, video graphics adapter card, speaker, presence-sensitive screen, one or more USB interfaces, video and/or audio output interfaces, or any other type of device capable of generating tactile, audio, video, or other output. In some examples, the output devices of user interface 59 may include a presence-sensitive display that may serve as a user interface device that operates both as one or more input devices and one or more output devices. Additional detail regarding an example of user interface 59 is described with respect to FIGS. 7A and 7B below.

In accordance with the techniques of the disclosure, user interface 59 presents a representation of the plurality of electrodes 48 of electrical stimulator 4. User interface 59 presents, e.g., a graphical representation of the therapy parameters, including amplitude, pulse-width, and frequency. User interface 59 may provide an input configured to receive selection of the initial set of therapy parameters or one of the one more additional sets of therapy parameters for electrodes 48. User interface 59 may provide fillable fields, or other adjustment input devices, such as increase or decrease input keys, that allow a user to input a desired value for a therapy parameter. User interface 59 may facilitate switching between different sets of therapy parameters or selecting different values for one or more parameters. Processing circuitry 53 may receive, from the user via user interface 59, an input specifying adjustments to the values of the initial parameters or secondary parameters or receive an input specifying an increase or decrease in the corresponding value of the parameter displayed on user interface 59. In some examples, user interface 59, when displaying the initial set of therapy parameters, may provide recommendations or suggestions for the secondary set of therapy parameters. For example, user interface 59 may display the initial set of therapy parameters with solid lines and/or in one color and may display suggested parameters in dashed lines or in another color. User interface 59 may be configured to display a confirmation input or otherwise receive a confirmation input from the user the confirms the selection of the recommended secondary set of therapy parameters instead of the initial set of therapy parameters. In some examples, user interface 59 may present an indication of the power consumption of the available therapy parameter sets, e.g., a total power required for each parameter set, a relative power consumption when compared with the initial set of therapy parameters (e.g., a percentage of the power consumption of the initial parameter set), a longevity increase (or decrease) with respect to the initial set of parameters, or any other indication of the power consumption of battery longevity differences between the different sets of parameters.

In operation, processing circuitry 53 may receive, from the user via user interface 59, a request to adjust one or more of therapy parameters corresponding to each particular electrode 48. For example, processing circuitry 53 may receive an input specifying an increase or decrease in a value of the one or more therapy parameters displayed on the user interface of programmer 40. Processing circuitry 53 transmits, via telemetry circuitry 58, the specified individual therapy parameters to electrical stimulation 4 to control electrical stimulator 4 electrical stimulation therapy according to respective therapy parameters of each electrode 48 according to the initial set of therapy parameters or the secondary set of parameters.

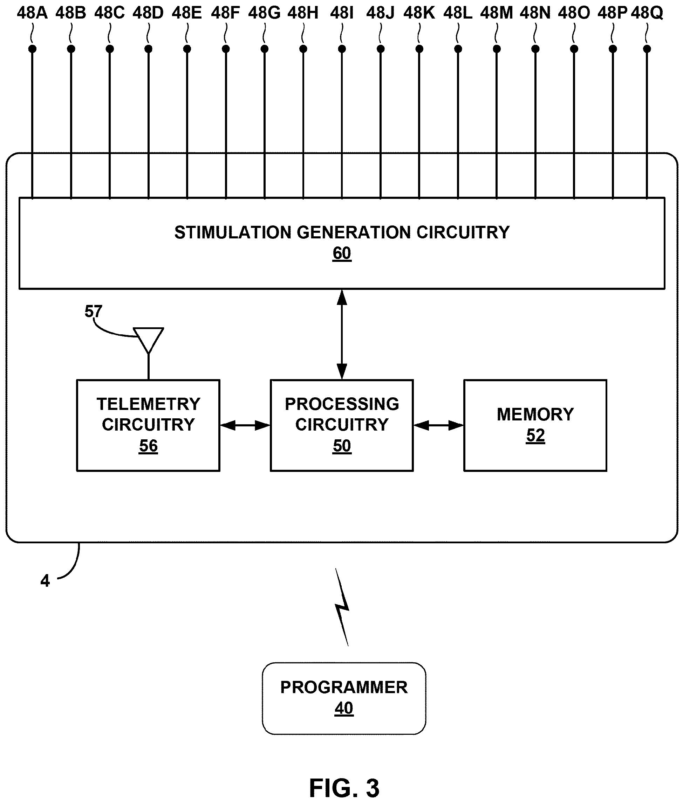

FIG. 3 is a block diagram illustrating example electrical stimulator 4 of FIG. 1 in further detail. In some examples, electrical stimulator 4 includes processing circuitry 50, memory 52, telemetry circuitry 56, antenna 57, and stimulation generation circuitry 60. Stimulation generation circuitry 60 is also shown in FIG. 3 coupled to electrodes 48A-Q (collectively "electrodes 48"). In some examples, electrodes 48A-48P may be implantable and may be deployed on one or more leads 12. With respect to FIG. 1, leads 12A and 12B may carry electrodes 48A-H and electrodes 48I-P, respectively. In some cases, one or more additional electrodes may be located on or within the housing of electrical stimulator 4, e.g., to provide a common or ground electrode or a housing anode. In some examples, a lead or lead carries eight electrodes to provide a 2x8 electrode configuration (two leads with 8 electrodes each), providing a total of sixteen different electrodes.

In some examples, different electrode configurations comprising a single lead, two leads, three leads, or more may be provided. In addition, electrode counts on leads may vary and may be the same or different from a lead to lead. Examples of other configurations include one lead with eight electrodes (1x8), one lead with 12 electrodes (1x12), one lead with 16 electrodes (1x16), two leads with four electrodes each (2x4), three leads with four electrodes each (3x4), three leads with eight electrodes each (3x8), three leads with four, eight, and four electrodes, respectively (4-8-4), two leads with 12 or 16 electrodes (2x12, 2x16), two or more leads with 11 or 13 electrodes, or other configurations. Processing circuitry 50 may select different electrodes to form various electrode combinations. In addition, processing circuitry 50 may assign various polarities to the selected electrodes to designate the electrodes as anodes or cathodes and form additional electrode configurations therefrom. Fewer or greater electrodes may be controlled by electrical stimulator 4 in other examples. For example, stimulation generation circuitry 60 may be coupled to 16 electrodes, 8 electrodes on each of two leads. For example each lead may include two ring electrodes and two levels of three electrodes at different circumferential positions around the lead perimeter.

Electrode 48Q represents one or more electrodes that may be carried on a housing of electrical stimulator 4. Electrode 48Q may also be a dedicated short lead extending from the housing, or a proximal portion of one of the leads carrying electrodes 48A-48P. The proximal portion may be closely adjacent to the housing, e.g., at or near a point at which a lead is coupled to the housing. Electrode 48Q may be configured as a regulated or unregulated electrode for use in an electrode configuration with selected regulated and/or unregulated electrodes among electrodes 48A-48P, which may be located on a lead body of one or more leads, as described above. Electrode 48Q may be formed together on a housing that carries the electrode and houses the components of electrical stimulator 4, such as stimulation generation circuitry 60, processing circuitry 50, memory 52, and telemetry circuitry 56.

Housing electrode 48Q may be configured for use as an anode to source current substantially simultaneously with one or more electrodes 48A-48P configured for use as cathodes sinking current in a unipolar arrangement. By way of specific example, electrodes 48A, 48B, and housing electrode 48Q each could be configured for use as anodes. Electrodes 48A, 48B could deliver electrical stimulation current substantially simultaneously with the electrical stimulation current delivered via housing electrode 48Q. In this illustration, one or more cathodes could be formed with other electrodes (e.g., any of electrodes 48C-48P) on the leads to sink current sourced by anodes 48A, 48B and 48Q.

Memory 52 may store instructions for execution by processing circuitry 50, stimulation therapy data, sensor data, and/or other information regarding therapy for patient 6. Processing circuitry 50 may control stimulation generation circuitry 60 to deliver stimulation according to a selected one or more of a plurality of programs or program groups stored in memory 52. Memory 52 may include any electronic data storage media, such as RAM, ROM, EEPROM, NVRAM, flash memory, magnetic memory, or the like. Memory 52 may store program instructions that, when executed by processing circuitry 50, cause the processing circuitry to perform various functions ascribed to processing circuitry 50 and electrical stimulator 4 in this disclosure.

Processing circuitry 50 may include one or more microprocessors, DSPs, ASICs, FPGAs, or other digital logic circuitry. Processing circuitry 50 controls operation of electrical stimulator 4. For example, processing circuitry 50 may control stimulation generation circuitry 60 to deliver stimulation therapy according to a selected program or group of programs retrieved from memory 52. In some examples, processing circuitry 50 may control stimulation generation circuitry 60 to deliver electrical signals, e.g., as stimulation pulses or continuous waveforms, with current amplitudes, pulse widths (if applicable), and rates specified by one or more stimulation programs. Processing circuitry 50 may also control stimulation generation circuitry 60 to selectively deliver stimulation via subsets of electrodes 48, also referred to as electrode combinations, and with polarities specified by one or more programs. The functions attributed to processing circuitry 50 herein may be embodied as software, firmware, hardware or any combination thereof.

Stimulation generation circuitry 60 is electrically coupled to electrodes 48A-P via conductors of the respective lead, such as lead 12 in FIG. 1. Stimulation generation circuitry 60 may be electrically coupled to one or more housing electrodes 48Q via an electrical conductor disposed within the housing of electrical stimulator 4. Housing electrode 48Q may be configured as a regulated or unregulated electrode to form an electrode configuration in conjunction with one or more of electrodes 48A-48P. Housing electrode 48Q may be configured for use as an anode to source current substantially simultaneously with one or more electrodes, e.g., any of electrodes 48A-48P, on one or more leads configured for use as anodes.

Stimulation generation circuitry 60 may include stimulation generation circuitry to generate stimulation pulses or waveforms and circuitry. Stimulation generation circuitry 60 produces an electrical stimulation signal in accordance with a set of therapy parameters through control signals from processing circuitry 50.

In one example implementation, stimulation generation circuitry 60 may be configured to deliver stimulation using one or more of electrodes 48A-P and housing electrode 48Q as stimulation electrodes, e.g., anodes. The anodes on the lead(s) and the housing may be used to deliver stimulation in conjunction with one or more cathodes on the lead(s). As one illustration, electrodes selected for delivery of stimulation current may comprise a housing anode, and anode on a lead, and a cathode on the same lead or a different lead. In other examples, the electrodes may include multiple anodes and/or multiple cathodes on one or more leads in conjunction with at least one anode on housing 14. In some examples, the electrodes may include one or more anodes on one or more leads, and one or more cathodes on the same lead or a different lead, e.g., a bipolar/multipolar arrangement.

Telemetry circuitry 56 may include a RF transceiver to permit bi-directional communication between electrical stimulator 4 and programmer 40. Telemetry circuitry 56 may include an antenna 57 that may take on a variety of forms. For example, antenna 57 may be formed by a conductive coil or wire embedded in a housing associated with medical device 4. In some examples, antenna 57 may be mounted on a circuit board carrying other components of electrical stimulator 4 or take the form of a circuit trace on the circuit board. In this way, telemetry circuitry 56 may permit communication with programmer 40 in FIG. 1, to receive, for example, new programs or program groups, or adjustments to programs or program groups. Telemetry circuitry 56 may be similar to telemetry circuitry 58 of programmer 40.

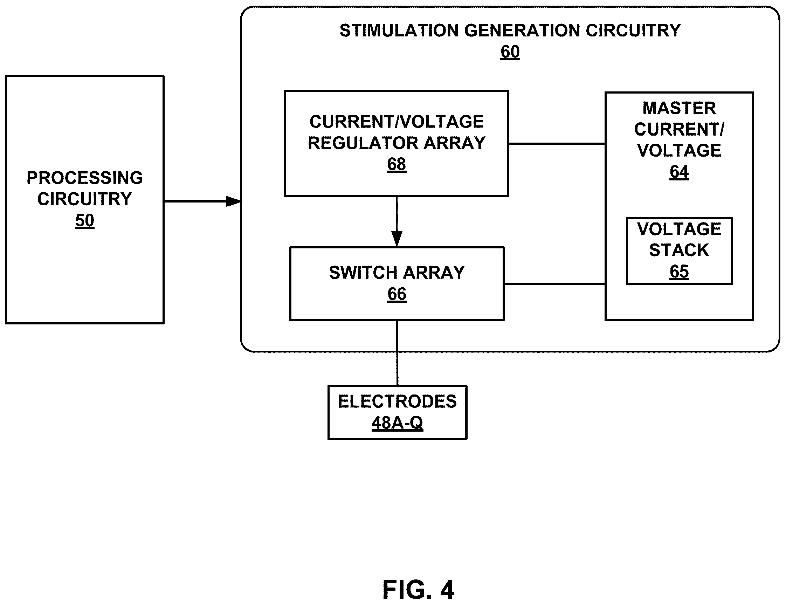

FIG. 4 is a block diagram illustrating an example of electrical stimulation generation circuitry 60 of electrical stimulator 4 of FIG. 3 in further detail. Stimulation generation circuitry 60 may be used with an electrical stimulator, e.g., to perform the functions of stimulation generation circuitry 60 as described with reference to FIG. 3. In the example of FIG. 4, stimulation generation circuitry 60 is selectively configured to deliver current stimulation pulses to patient 6 via electrodes 48. However, this disclosure is not limited to examples in which regulated current pulses are delivered. In other examples, stimulation generation circuitry 60 may provide continuous, regulated current waveforms, rather than regulated current pulses. In some examples, stimulation generation circuitry 60 may deliver combinations of continuous waveforms and pulses, or selectively deliver either continuous waveforms or pulses. Stimulation generation circuitry 60 may generate either constant current-based or constant voltage-based stimulation in the form of pulses or continuous waveforms. Stimulation generation circuitry 60 may also be controlled to provide constant power (current-voltage product) or controlled charge stimulation pulses.

In the example illustrated in FIG. 4, stimulation generation circuitry 60 includes master current/voltage 64, and current/voltage regulator array 68. In some examples, stimulation generation circuitry 60 may further include a switch array 66. Master current/voltage 64 may provide operating power to current/voltage regulator array 68, and may include a regulated current or regulated voltage that sets the level of the master current (e.g., master electrical current amplitude) or master voltage. As shown in FIG. 4, master current/voltage 64 may be coupled to provide operating power for the current/voltage regulator array 68 and provide a master current, or master voltage when appropriate, for connection to electrodes 48. The maximum operating current level and the master current level provided to regulate current regulator array 68 may be different at any given time. For example, a master electrical current amplitude may be less than the maximum operating current level, such that the master electrical current amplitude may be increased or decreased according to minimum and maximum operating conditions.

Processing circuitry 50 may control (e.g., via a stimulation controller) switch array 66 and current/voltage regulator array 68 to deliver stimulation via electrodes 48. In operation, processing circuitry 50 may control delivery of electrical stimulation according to one or more programs that may specify stimulation parameters such as electrode combination, electrode polarity, stimulation current amplitude, pulse rate, and/or pulse width as well as the percentage of source current distributed among or contributed by a housing anode and one or more lead anodes on one or more leads, and the percentage of sink current sunk by one or more cathodes. Programs may be defined by a user via an external controller and downloaded to an electrical stimulator 4.

Current/voltage regulator array 68 includes a plurality of regulated current sources or sinks. A current regulator may function as either a current source or sink, or be selectively configured to operate as either a source or a sink. In some examples, current/voltage regulator array 68 may regulate voltage instead of, or in addition to, current. For convenience, the term "current regulator" may be used in some instances to refer to either a source or sink. Hence, each of the current regulators in current/voltage regulator array 68 may operate as a regulated current source that delivers stimulation via a corresponding one of electrodes 48 or a regulated current sink that receives current from a corresponding one of electrodes 48, where electrodes 48 may be provided on leads, on a stimulator housing, on a leadless stimulator, or in other arrangements. Although multiple current sources or sinks are described herein, electrical stimulator 4 may include a single current source or sink in other examples and still support locking multiple electrodes into a relationship having a ratio of values for one or more therapy parameters.

In examples including switch array 66, each switch of switch array 66 may couple a corresponding one of electrodes 48 to either a corresponding bi-directional current regulator of current/voltage regulator array 68 or to master current/voltage 64. In some examples, processing circuitry 50 selectively opens and closes switches in switch array 66 to configure a housing electrode (e.g., electrode(s) 48Q), and one or more of electrodes 48A-48P on one or more leads as regulated electrodes by connection to regulated current sources or sinks in current/voltage regulator array 68. In some examples, processing circuitry 50 may selectively open and close switches in switch array 66 to configure either the housing electrode, e.g., electrode 48Q, or an electrode on the lead as an unregulated electrode by connection to master current/voltage 64. In addition, processing circuitry 50 may selectively control individual regulated current sources or sinks in current/voltage regulator array 68 to deliver stimulation current pulses to the selected electrodes. In examples where switch array 66 is not used, electrodes 48 may nevertheless be coupled to current/voltage regulator array 68 and/or to master current/voltage 64.

Master current/voltage 64 may be a high or low voltage supplied by a regulated power source, depending on whether an electrode is programmed to be an unregulated source (high voltage rail) or unregulated sink (low voltage rail). Hence, master current/voltage 64 may produce high and low master current, or master voltages when appropriate, for selective coupling to unregulated, reference electrodes as needed. A regulated power source may produce one or more regulated voltage levels for use as master current/voltage 64 and for use as a power rail for current/voltage regulator array 68. Although the same master current/voltage 64 is shown as being coupled to current/voltage regulator array 68 in FIG. 4, different current amplitude may be used for the master current coupled to switch array 66 and the maximum current amplitude provided to current regulator array 68. In any event, a regulated power source may generate the regulated current amplitudes from current provided by a power source or multiple power sources, such as one or more batteries (e.g., rechargeable or non-rechargeable batteries).

In the illustrated example, master current/voltage 64 includes voltage stack 65. Voltage stack 65 outputs a voltage that is a multiple of the battery voltage that us used to supply current/voltage regulator array 68. The multiple of the battery voltage provided by voltage stack 65 is based on a driving frequency provided by processing circuitry 50. Generally, the higher the driving frequency, the higher the multiplier. Additionally, higher battery multipliers are less energy efficient. Further, greater the differences between voltage supplied to current/voltage regulator array 68 (e.g., 4.5V by voltage stack 65, etc.) and the voltage supplied by current/voltage regulator array 68 to drive electrodes 48 (e.g., 3.9V, etc.) are less energy efficient. Voltage stack 65 provide discrete steps or tiers (e.g., 1.25, 1.50, 1.75, etc.) of multiples of battery voltage based on the driving frequency. For example, at one driving frequency, voltage stack 65 may provide 1.25 times battery voltage, and at the next available driving frequency, voltage stack 65 may provide 1.50 times battery voltage. For example, for a 3V battery, voltage stack 65 may provide 4.50V when driven at a first frequency (sometimes referred to as a "4.50V voltage tier") and may provide 3.75V when driven at a second frequency (sometimes referred to as a "3.75V voltage tier."). Example amplitudes and their corresponding battery multipliers are illustrated on Table 1 below.

**Table 1**

| Amplitude (mA) | Stack Multiplier | Stack Output Voltage (V) |
|---|---|---|
| 0.2 | 1.25 | 3.75 |
| 0.6 | 1.25 | 3.75 |
| 1 | 1.25 | 3.75 |
| 1.4 | 1.25 | 3.75 |
| 1.8 | 1.25 | 3.75 |
| 2.2 | 1.25 | 3.75 |
| 2.6 | 1.5 | 4.50 |
| 3 | 1.5 | 4.50 |
| 3.4 | 1.75 | 5.25 |
| 3.8 | 2 | 6.00 |
| 4.2 | 2.25 | 6.75 |
| 4.6 | 2.5 | 7.50 |
| 5 | 2.5 | 7.50 |

These voltage tiers determined by the battery voltage and battery voltage multiplier may define thresholds that system 2 uses to determine the secondary set of therapy parameters. In some examples, the secondary set of therapy parameters are selected such that voltage stack 65 produces voltage at a lower voltage tier. For example, if the initial set of therapy parameters result in a voltage to generate the specified amplitude that falls between 3.75V and 4.50V tiers of voltage stack 65, the secondary set of therapy parameters may be selected that results in a voltage to generate the specified amplitude that falls below 3.75V However, the system may also change one or more parameters of the secondary set of therapy parameters to accommodate the reduction in voltage and maintain similar electrical field deliver to the patient. For example, processing circuitry 50 may increase the pulse width or adjust the frequency of one or more pulse trains in the stimulation to enable the reduction in stack multiplier.

Processing circuitry 50 controls the operation of switch array 66 to produce electrode configurations defined by different stimulation programs. In some cases, the switches of switch array 66 may be metal-oxide-semiconductor field-effect-transistors (MOSFETs) or other circuit components used for switching electronic signals. The switches of switch array 66 may be designed to carry an amount of unregulated current that may be coupled to a corresponding electrode through an unregulated current path associated with master current/voltage 64. In some examples, two or more regulated electrodes 48 may be intentionally programmed to deliver different amounts of current, such that the regulated electrodes produce an unbalanced current distribution. In other examples, regulated source and sink current may be balanced such that substantially all current may be sourced and sunk via respective regulated current sources and sinks.

To provide individual control of electrodes 48 as either regulated electrodes or as unregulated, reference electrodes, processing circuitry 50 controls operation of switch array 66 and current/voltage regulator array 68. When stimulation is delivered to patient 6, for the example of current pulses, processing circuitry 50 controls switch array 66 to couple selected stimulation electrodes for a desired electrode combination to respective current regulators of current/voltage regulator array 68 or to master current/voltage 64, as needed. Processing circuitry 50 controls the regulated bi-directional current sources of current/voltage regulator array 68 coupled to regulated electrodes to source or sink specified amounts of current. For example, processing circuitry 50 may control selected current sources or sinks on a pulse-by-pulse basis to deliver current pulses to corresponding electrodes.

Processing circuitry 50 also deactivates the regulated bi-directional current regulators of current/voltage regulator array 68 tied to inactive electrodes, e.g., electrodes that are not active as regulated electrodes in a given electrode configuration. Each regulated bidirectional current regulator of current/voltage regulator array 68 may include an internal enable switch controlled by processing circuitry 50 that disconnects regulated power from the current regulator or otherwise disables the current source when the corresponding electrode is not used as a regulated electrode.

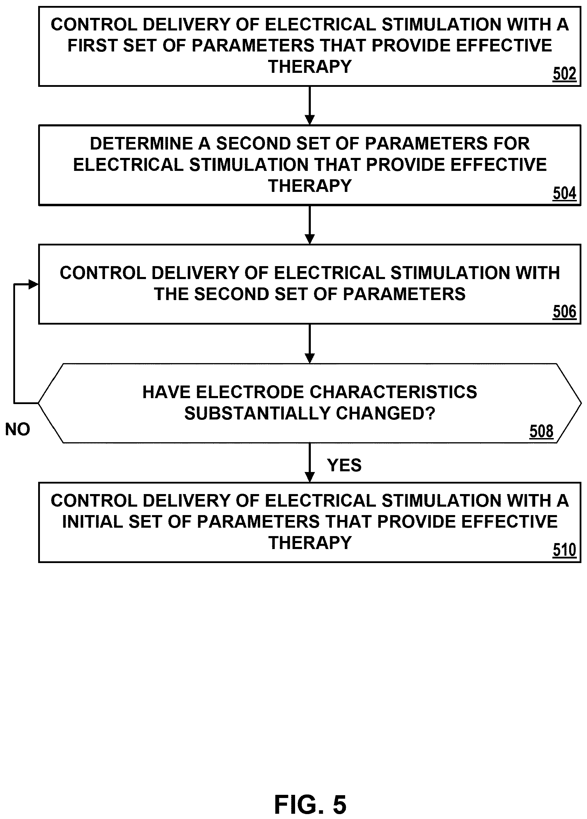

FIG. 5 is a flowchart of an example method to control delivery of electrical stimulation in accordance with the techniques of this disclosure. Initially, processing circuity 50 receives or determines an initial set of therapy parameters (502). For examples, system 2 may determine the initial set of therapy parameters that deliver effective therapy based on clinical data and patient feedback. In some examples, processing circuitry 50 may control delivery of electrical stimulation with the initial set of therapy parameters. Processing circuitry then determines, based on a lower voltage stack setting, a secondary set of therapy parameters that provide the effective therapy (504). For example, system 2 may determine the secondary set of therapy parameters that uses, for example, a lower voltage tier of voltage stack 65 based on a strength duration curve that predicts substantially equivalent therapy at different amplitudes and pulse widths based on characteristics of the patient. As another example, processing circuity 50 may determine a total charge or charge density provided by the initial set of therapy parameters and select an amplitude-pulse width pair that provides substantially similar (e.g., ±10%, etc.) charge or charge density with a lower amplitude. As another example processing circuity 50may determine the secondary set of therapy parameters based on VOA models of the initial set of therapy parameters.

Processing circuity 50 may determine the secondary set of therapy parameters in response to receiving an input that causes an increase to the stack voltage (e.g., a parameter value change that requires an increase in the multiplier provided by voltage stack 65, etc.). For example, after the initial set of therapy parameters is determined, processing circuity 50 may at a later time determine an alternate set of therapy parameters to readjust the effective therapy. However, processing circuitry 50 may determine that the new parameter values (e.g., a higher amplitude) require using a higher voltage stack multiplier. In response to a set of parameters being selected that causes the voltage multiplier of voltage stack 65 to increase, processing circuity 50 may determine a secondary set of therapy parameters (e.g., a set of therapy parameters with a lower amplitude and a longer pulse width, etc.) that results in a voltage to generate the specified amplitude that is at least within the same voltage tier as the original set of therapy parameters. As another example, the available battery voltage may drop over time as the battery discharges. Eventually, this may cause the electrical stimulator 4 to use a higher stack multiplier to compensate for the lost voltage. In some examples, processing circuitry 50 may determine a secondary set of therapy parameters (e.g., a set of therapy parameters with a lower amplitude and a longer pulse width, etc.) that results in a voltage to generate the specified amplitude that is at least within the same voltage tier as the original set of therapy parameters. Alternatively or additionally, in some examples, processing circuitry 50 may from time-to-time determine a set of therapy parameters such that results in a voltage to generate the specified amplitude that is at least within the same voltage tier as the original set of therapy parameters before a higher voltage stack multiplier is required. Alternatively or additionally, in some examples, processing circuitry 50 may, from time to time, determine a secondary set of therapy parameters without an explicit trigger. For example, processing circuitry50 may periodically determine a secondary set of therapy parameters (e.g., determine a more power efficient set of therapy parameters over time to conserve battery life, etc.).

Processing circuity 50 controls delivery of electrical stimulation with the secondary set of parameters (506). In some examples, processing circuitry 50 may request confirmation before controlling delivery of electrical stimulation with the secondary set of parameters (e.g., via a patient programmer device, etc.) Processing circuity 50determines whether characteristics of electrodes 48 have substantially changed (508). For example, changing characteristics of stimulated tissue may cause impedances of respective current paths that may include lead conductors, electrodes, and tissue between electrodes to change over time such that the initial set of therapy parameters may be more effective to provide therapy. When the characteristics of electrodes 48 have substantially changed ("YES" branch at 508), electrical stimulator 4 controls delivery of electrical stimulation with a different set of therapy parameters (e.g., the initial set of therapy parameters, etc.) to provide effective therapy (510). When the characteristics of electrodes 48 have not substantially changed ("NO" branch at 508), Processing circuity 50 continues to control delivery of electrical stimulation with the secondary set of therapy parameters to provide effective therapy (506).

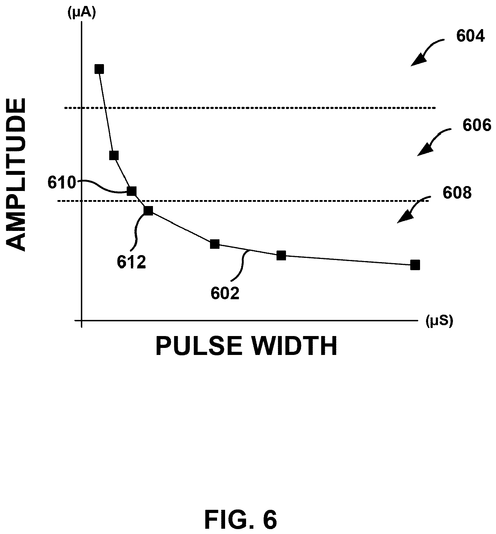

FIG. 6 illustrates example strength-duration curve 602. For example, strength-duration curve 602 may represent strength-duration curves representative of characteristics of appropriate tissue sites for a patient or between different patients. In the illustrated examples, the strength-duration curves 602A falls within a first voltage tier 604(e.g., a voltage generated by voltage stack 65 using a relatively higher multiplier) a second voltage tier 606 (e.g., a voltage generated by voltage stack 65 using a relatively lower multiplier), and a third tier 608 (e.g., a voltage generated by voltage stack 65 using a relatively lower multiplier). Initial set of parameters 610 may fall on strength-duration curve 602 within the second voltage tier 606 where the voltage to produce the amplitude is near the third tier 608. Processing circuity 50 may select secondary set of parameters 612 on strength-duration curve 602 that (a) falls within second voltage tier 608 and, in some examples, is near the top range of second voltage tier 608 (e.g., to minimize head room between the voltage to drive the amplitude of secondary set of parameters 612 and the voltage provided by second voltage tier 610, etc.). In the illustrated example, secondary set of parameters 612 has a longer pulse width than the initial set of parameters 610. In this manner, the second voltage tier 608 may consume less energy than the higher voltage tier 606 while providing a similar charge to the tissue.

FIGS. 7A and 7B are conceptual diagrams illustrating screens of an example user interface 700 in accordance with the techniques of the disclosure. FIG. 7A is a conceptual diagram illustrating a screen with an initial set of therapy parameters. FIG. 7B is a conceptual diagram illustrating a screen with a secondary set of therapy parameters. User interface 700 may be an example of user interface 59 of programmer 40 of FIG. 2.

User interface 700 depicts a representation of electrode icons 748A-748D (hereinafter, "electrode icons 748") disposed on lead icon 712 within display window 720. In the example of FIGS. 7A-7B, each of electrode icons 748A-748D correspond to a respective one of electrodes 48A-48D of FIGS. 1 and 3, and lead icon 712 corresponds to lead 12 of FIG. 1. In the example of FIG. 7A, electrode icons 748D and 748B indicate that electrodes 48D and 48B are selected to act as cathodes and electrode icon 748C indicates that electrode 48C is selected to act as an anode. Display window 720 further depicts a representation of a predicted VOA 702A generated by delivery of electrical stimulation by electrical stimulator 4 according to initial set of therapy parameters selected for electrodes 48B, 48C, and 48D. However, display window 720 may display one or more representations to assist the clinician to compare sets of therapy parameters. For example, display window 720 may additionally or alternatively, display a strength-duration curve (e.g., similar to the strength-duration curves 600 and 602 of FIG. 6) selected based on patient characteristics. Display window 720 may additionally or alternatively, display a representation of a charge density or a predicted electrical field to facilitate selecting the secondary set of therapy parameters to provide the effective therapy. The charge density or electric field may be displayed instead of, or in addition to, VOA 702A.

User interface 700 includes a toggle button 738 that allows a clinician to activate or deactivate delivery of electrical stimulation by electrical stimulator 4 according to therapy parameters selected for electrodes 48B, 48C, and 48D. In the illustrated example, user interface 700 includes a selection menu 739 to select between displaying information regarding different sets of parameters (e.g., the initial set of parameters and the secondary set of parameters, etc.).User interface 700 further includes therapy parameter control panel 722 that allows a clinician to adjust values of therapy parameters for a currently-selected electrode 48. In the example of FIG. 7A, the clinician has selected electrode icon 748C and set a value of 2.9mA for a current amplitude of electrical stimulation delivered via electrodes 48. The user may adjust the value of the therapy parameter for electrodes 48 by pressing incremental increase button 732 or incremental decrease button 734. In this example, incremental increase button 732 and incremental decrease button 734 adjust the value of the therapy parameter by 0.1 milliamps. Further, the clinician may adjust the value of the therapy parameter for electrode 48C to a maximum value by pressing maximum button 730 or a minimum value by pressing minimum button 736. The maximum or minimum values may be predetermined based on patient comfort and stimulation efficacy or predetermined based on system limits. Alternatively or in addition, the user may proportionally adjust the stimulation field of the entire active electrode level using the amplitude slider. The clinician may select the type of therapy parameter (e.g., current amplitude, pulse duration, or pulse frequency) by selecting a corresponding therapy parameter type button such as milliamp button 740, pulse width button 742, or pulse frequency button 744. In the illustrated example of FIG. 7A, pulse width button 742 indicates that the initial set of therapy parameters has a pulse width parameter of 60 microseconds (µs).

In the example of FIG. 7B, user interface 700 depicts a representation of electrodes 48 providing electrical stimulation based on the secondary set of therapy parameters. Display window 720 further a representation of a VOA 702B generated by delivery of electrical stimulation by electrical stimulator 4 according to secondary set of therapy parameters selected for electrodes 48B, 48C, and 48D. In the example of FIG. 7A, the clinician has selected electrode icon 748C and set a value of 2.9mA for a current amplitude of electrical stimulation delivered via electrodes 48. Additionally, pulse width button 742 indicates that the initial set of therapy parameters has a pulse width parameter of 90µs. System 2 may select these therapy parameters because, for example, VOA 702B is substantially similar to VOA 702A.

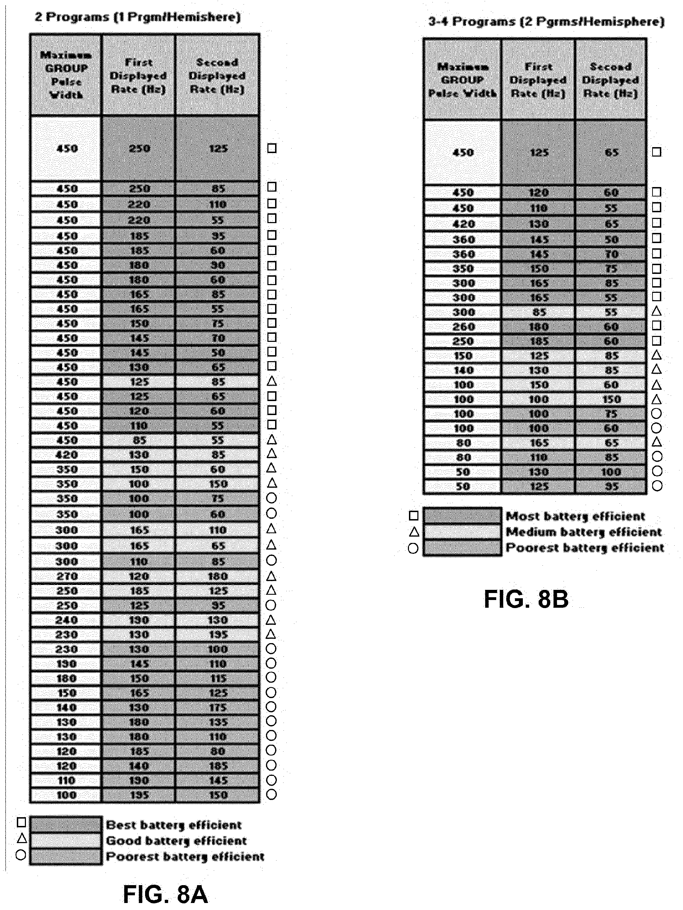

FIGS. 8A and 8B are conceptual diagrams illustrating example battery efficiencies for different pulse rates at various pulse widths. The example of FIG. 8A provides example parameter options and estimated battery efficiencies for different pulse widths and pulse frequencies (e.g., pulse rate) for two different stimulation programs defining stimulation to different respective hemispheres of the brain. Generally, battery efficiency is greater for longer pulse widths and higher pulse rates. In addition, the battery efficiency is better for the same pulse widths when the common frequency for driving the different pulse rates is lower. For example, the common frequency of programs with a pulse rate of 130 Hz and 65 Hz is 130 Hz. The common frequency of programs with a pulse rate of 125 Hz and 85 Hz is approximately 250 Hz, which results in reduced battery efficiency when compared with the 130 Hz common frequency of the prior programs. The system or user can then select program pulse rate combinations, and/or pulse widths, based at least in part on which pulse rates and pulse width combinations improve battery efficiency and longevity. In the legend which applies to the diagram of FIGS. 8A, squares indicate the best battery efficient configurations, triangles indicate good battery efficient configurations (e.g., less efficient than the best configurations), and circles indicate the poorest battery efficient configurations.

The example of FIG. 8B provides example parameter options and estimated battery efficiencies for different pulse widths and pulse frequencies (e.g., pulse rate) for three or four different stimulation programs defining stimulation to different respective hemispheres of the brain. Similar relationships occur as described with respect to FIG. 8A, but fewer pulse width and pulse rate options may be available with a greater number of programs with different pulse rates. The system can calculate the relative battery efficiencies for the different stimulation parameter options based on calculations of the system that may include the current amplitudes, resistance of the circuit, capacitance, interphase delay between phases of the same pulse, voltage stack settings, batter voltage, etc. In the legend which applies to the diagram of FIGS. 8B, squares indicate the most battery efficient configurations, triangles indicate medium battery efficient configurations, and circles indicate the poorest battery efficient configurations.

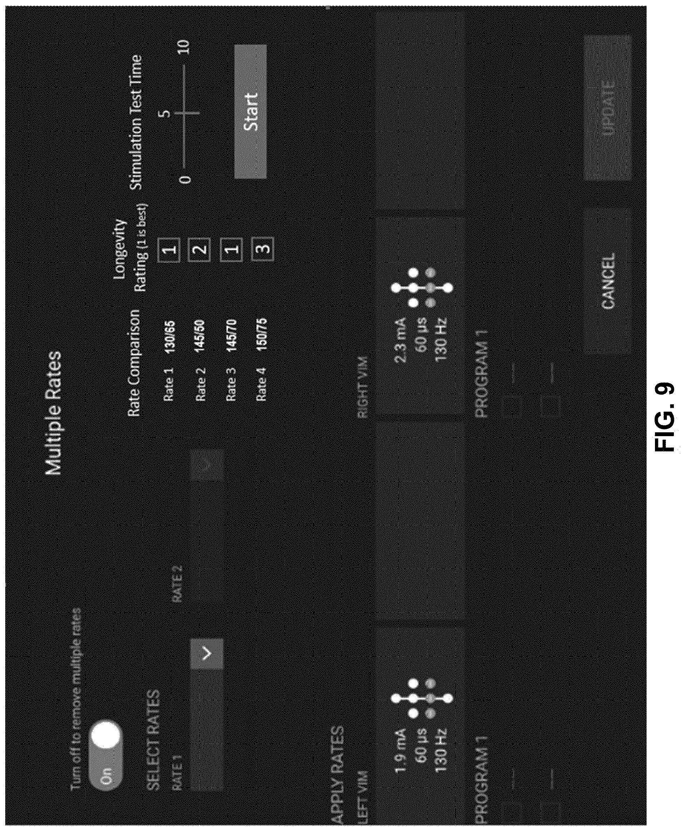

FIG. 9 illustrates an example user interface that provides estimated longevity information for different pulse rates of electrical stimulation. As discussed with respect to FIGS. 8A and 8B, pulse trains with different pulse frequencies may change the efficiency of battery consumption due to various system factors. As shown in FIG. 9, the user interface may include input fields for the user to select multiple pulse rates for respect stimulation pulse trains. In response the user selecting the first pulse rate (e.g., rate 1), the system may automatically limit the options from the pull down menu for the second pulse rate (e.g., rate 2) to those pulse rates that the system can produce concurrently with the first pulse rate (e.g., rates that have a common rate achievable by the system). These pulse trains may be delivered to the same or different hemispheres, as shown with the current amplitude, pulse width, frequency, and electrode combination program fields.

In addition, FIG. 9 illustrates that the user interface can provide longevity information for different selectable pulse rate combinations. As shown, the system may generate a longevity rating that estimates the relative efficiency of the battery using each pulse rate combination, such as 130 Hz and 65 Hz for rate 1 and 145 Hz and 50 Hz for rate 2. The user, or system, can then select the pulse rates for each program using this longevity information. The user interface may also include a stimulation test field that is configured to receive user input selecting a desired time to test the programs with the selected pulse rates to see how the patient responds to the stimulation. The system may start the stimulation test in response the user selecting the start button and run the test for the duration of the selected test time shown in the time graph. The user interface may also receive user input requesting cancelation of the stimulation test at any time. Although the user interface of FIG. 9 provides estimated longevity information for different pulse rates, the user interface may additionally or alternatively provide longevity information for different selectable pulse widths and/or amplitudes. As described herein, these different stimulation parameters may enable system 2 to utilize different voltage stack settings or other system settings in order to improve battery efficiency and/or reduce power consumption while maintaining desired therapy efficiency from stimulation.

The following examples are described herein.

Example 1: A system includes a stimulation generator includes receive a first set of parameter values that use a first stack voltage of the voltage stack to provide a first electrical stimulation defining a first therapy; determine, based on a second stack voltage lower than the first stack voltage, a second set of parameter values that define a second electrical stimulation, the second set of parameters defining a lower amplitude of electrical stimulation; and control the stimulation generator to deliver the second electrical stimulation according to the second set of parameter values using the second stack voltage of the voltage stack.

Example 2: The system of example 1, wherein the first set of parameter values comprise a first pulse width and the second set of parameter values comprises a second pulse width, and wherein the second pulse width is greater than the first pulse width.

Example 3: The system of any of examples 1 and 2, wherein to control the stimulation generator according to the first set of parameter values, the processing circuitry is configured to drive the voltage stack with a first frequency and wherein to control the stimulation generator according to the second set of parameter values, the processing circuitry is configured to drive the voltage stack with a second frequency lower than the first frequency.

Example 4: The system of any of examples 1 through 3, wherein the first electrical stimulation provides a first charge and the second electrical stimulation provides a second charge, and wherein the processing circuitry is configured to select the second set of parameter values to generate the second electrical stimulation having the second charge within a target tolerance of the first charge of the first electrical stimulation.

Example 5: The system of any of examples 1 through 4, wherein the processing circuitry is configured to select the second set of parameters values based on a strength-duration curve defining a relationship between two parameters of the first set of parameter values.

Example 6: The system of any of examples 1 through 5, wherein the first electrical stimulation provides a first charge density and the second electrical stimulation provides a second charge density, and wherein the processing circuitry is configured to select the second set of parameter values to generate the second electrical stimulation having the second charge density within a target tolerance of the first charge density of the first electrical stimulation.

Example 7: The system of any of examples 1 through 6, wherein the processing circuitry is configured to determine the second set of parameters in response to receiving a command requesting an amplitude that requires a stack voltage greater than the second stack voltage.

Example 8: The system of any of examples 1 through 7, wherein the processing circuitry is configured to determine the second set of parameters in response to a reduction the battery voltage that requires an increase to the stack voltage greater than the second stack voltage to maintain the amplitude specified by the first set of parameter values.

Example 9: The system of any of examples 1 through 8, wherein the processing circuitry is further configured to: monitor an impedance of one of more electrodes that deliver the electrical stimulation; detect an increase to the impedance of the one or more electrodes; and responsive to detecting the increase to the impedance, control the delivery of electrical stimulation according to the first set of parameters.

Example 10: The system of any of examples 1 through 9, wherein the apparatus further comprises a programming interface, and wherein the processing circuitry is further configured to, prior to controlling the delivery of the electrical stimulation according to the second set of parameters: transmit a message, via the programming interface, requesting user confirmation to deliver the second electrical stimulation according to the second set of parameter values; and responsive to receiving the confirmation, control the stimulation generator to deliver second electrical stimulation according to the second set of parameter values.

Example 11: The system of any of examples 1 through 10, wherein an implantable medical device comprises the stimulation generator, the processing circuitry, and a power storage device configured to provide the battery voltage level.

Example 12: A method includes receiving, by processing circuitry, a first set of parameter values that use a first stack voltage of a voltage stack to provide a first electrical stimulation defining a first therapy; determining, by the processing circuitry, based on a second stack voltage lower than the first stack voltage, a second set of parameter values that define a second electrical stimulation to provide the first therapy, the second set of parameter values defining a lower amplitude of electrical stimulation; and controlling, by the processing circuitry, a stimulation generator to deliver the second electrical stimulation according to the second set of parameter values using the second stack voltage of the voltage stack.

Example 13: The method of example 12, wherein the first set of parameter values comprises a first pulse width and the second set of parameter values comprises a second pulse width, and wherein the second pulse width is greater than the first pulse width.

Example 14: The method of any of examples 12 and 13, wherein controlling the stimulation generator according to the first set of parameters comprises driving the voltage stack with a first frequency and wherein controlling the stimulation generator according to the second set of parameters comprises driving the voltage stack with a second frequency lower than the first frequency.

Example 15: The method of any of examples 12 through 14, wherein the first electrical stimulation provides a first charge and the second electrical stimulation provides a second charge, and wherein selecting the second set of parameter values to generate the second electrical stimulation comprises selecting the second set of parameter values to generate the second electrical stimulation where the second charge is within a target tolerance of the first charge.

Example 16: The method of any of examples 12 through 15, wherein selecting the second set of parameters values based on a strength-duration curve defining a relationship between two parameters of the first set of parameter values.

Example 17: The method of any of examples 12 through 16, wherein the first electrical stimulation provides a first charge density and the second electrical stimulation provides a second charge density, and wherein selecting the second set of parameter values to generate the second electrical stimulation comprises selecting the second set of parameter values to generate the second electrical stimulation where the second charge density is within a target tolerance of the first charge density.

Example 18: The method of any of examples 12 through 17 includes transmitting a message, via a programming interface, requesting user confirmation to deliver the second electrical stimulation according to the second set of parameter values; and responsive to receiving the confirmation, controlling the stimulation generator to deliver second electrical stimulation according to the second set of parameter values.

Example 19: The method of any of examples 12 through 18 includes monitoring an impedance of one of more electrodes that deliver the electrical stimulation; and responsive to detecting the increase to the impedance of the one or more electrodes, determining a third set of parameter values that define a third electrical stimulation, the third set of parameter values defining a lower amplitude and longer pulse width of electrical stimulation compared to the first set of parameter values.

Example 20: A computer readable medium comprising instructions that, when executed, cause processing circuitry to receive a first set of parameter values that use a first stack voltage of a voltage stack to provide a first electrical stimulation defining a first therapy; determine, based on a second stack voltage lower than the first stack voltage, a second set of parameter values that define a second electrical stimulation to provide the first therapy, the second set of parameter values defining a lower amplitude of electrical stimulation; and control a stimulation generator to deliver the second electrical stimulation according to the second set of parameter values using the second stack voltage of the voltage stack.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A system comprising:
a stimulation generator comprising a voltage stack configured to provide a stack voltage based on a multiplier of a battery voltage; and
processing circuitry configured to:
receive a first set of parameter values that use a first stack voltage of the voltage stack to provide a first electrical stimulation defining a first therapy;
determine, based on a second stack voltage lower than the first stack voltage, a second set of parameter values that define a second electrical stimulation, the second set of parameters defining a lower amplitude of electrical stimulation; and
control the stimulation generator to deliver the second electrical stimulation according to the second set of parameter values using the second stack voltage of the voltage stack.

2. The system of claim 1, wherein the first set of parameter values comprise a first pulse width and the second set of parameter values comprises a second pulse width, and wherein the second pulse width is greater than the first pulse width.

3. The system of any of the preceding claims, wherein to control the stimulation generator according to the first set of parameter values, the processing circuitry is configured to drive the voltage stack with a first frequency and wherein to control the stimulation generator according to the second set of parameter values, the processing circuitry is configured to drive the voltage stack with a second frequency lower than the first frequency.

4. The system of any of the preceding claims, wherein the first electrical stimulation provides a first charge and the second electrical stimulation provides a second charge, and wherein the processing circuitry is configured to select the second set of parameter values to generate the second electrical stimulation having the second charge within a target tolerance of the first charge of the first electrical stimulation.

5. The system of any of the preceding claims, wherein the processing circuitry is configured to select the second set of parameters values based on a strength-duration curve defining a relationship between two parameters of the first set of parameter values.

6. The system of any of the preceding claims, wherein the first electrical stimulation provides a first charge density and the second electrical stimulation provides a second charge density, and wherein the processing circuitry is configured to select the second set of parameter values to generate the second electrical stimulation having the second charge density within a target tolerance of the first charge density of the first electrical stimulation.

7. The system of any of the preceding claims, wherein the processing circuitry is configured to determine the second set of parameters in response to receiving a command requesting an amplitude that requires a stack voltage greater than the second stack voltage.

8. The system of any of the preceding claims, wherein the processing circuitry is configured to determine the second set of parameters in response to a reduction the battery voltage that requires an increase to the stack voltage greater than the second stack voltage to maintain the amplitude specified by the first set of parameter values.

9. The system of any of the preceding claims, wherein the processing circuitry is further configured to:
monitor an impedance of one of more electrodes that deliver the electrical stimulation;
detect an increase to the impedance of the one or more electrodes; and
responsive to detecting the increase to the impedance, control the delivery of electrical stimulation according to the first set of parameters.

10. The system of any of the preceding claims, wherein the apparatus further comprises a programming interface, and wherein the processing circuitry is further configured to, prior to controlling the delivery of the electrical stimulation according to the second set of parameters:
transmit a message, via the programming interface, requesting user confirmation to deliver the second electrical stimulation according to the second set of parameter values; and
responsive to receiving the confirmation, control the stimulation generator to deliver second electrical stimulation according to the second set of parameter values.

11. The system of any of the preceding claims, wherein an implantable medical device comprises the stimulation generator, the processing circuitry, and a power storage device configured to provide the battery voltage level.

12. A computer readable medium comprising instructions that when executed, cause a system to:
receive a first set of parameter values that use a first stack voltage of a voltage stack to provide a first electrical stimulation defining a first therapy;
determine, based on a second stack voltage lower than the first stack voltage, a second set of parameter values that define a second electrical stimulation to provide the first therapy, the second set of parameter values defining a lower amplitude of electrical stimulation; and
control a stimulation generator to deliver the second electrical stimulation according to the second set of parameter values using the second stack voltage of the voltage stack.

13. The computer readable medium of claim 12, wherein the first set of parameter values comprise a first pulse width and the second set of parameter values comprises a second pulse width, and wherein the second pulse width is greater than the first pulse width.

14. The computer readable medium of any of the claims 12 or 13, wherein to control the stimulation generator according to the first set of parameter values, the processing circuitry is configured to drive the voltage stack with a first frequency and wherein to control the stimulation generator according to the second set of parameter values, the processing circuitry is configured to drive the voltage stack with a second frequency lower than the first frequency.

15. The computer readable medium of any of the claims 12 to 14, wherein the first electrical stimulation provides a first charge and the second electrical stimulation provides a second charge, and wherein the processing circuitry is configured to select the second set of parameter values to generate the second electrical stimulation having the second charge within a target tolerance of the first charge of the first electrical stimulation.
